Europäisches Patentamt

**European Patent Office**

Office européen des brevets

(11) Numéro de publication: **0 071 534**
**A1**

## (12) DEMANDE DE BREVET EUROPEEN

(21) Numéro de dépôt: **82401406.2**

(22) Date de dépôt: **28.07.82**

(51) Int. Cl.³: **G 01 K 7/02**
**H 01 R 23/26**

(30) Priorité: **30.07.81 FR 8114833**

(43) Date de publication de la demande:
**09.02.83 Bulletin 83/6**

(84) Etats contractants désignés:
**AT BE CH DE FR GB IT LI LU NL SE**

(71) Demandeur: **MECILEC**
**91 bis, rue du Cherche-Midi**
**F-75006 Paris(FR)**

(72) Inventeur: **Berger, Jean-Claude**
**36, route de Saint Ambroix**
**F-36100 Issoudun(FR)**

(72) Inventeur: **Hatchadour, Christian**
**17, rue des Jacinthes**
**F-36100 Issoudun(FR)**

(74) Mandataire: **Martin, Jean-Jacques et al,**
**Cabinet REGIMBEAU 26, Avenue Kléber**
**F-75116 Paris(FR)**

(54) **Pyromètre à immersion.**

(57) La présente invention concerne un pyromètre à immersion comprenant une sonde à immerger dans un milieu dont on veut mesurer à la fois la température et la teneur en oxygène. Cette sonde comprend un couple thermo-électrique platine/platine rhodié, une canne pour porter la sonde à distance pendant la mesure et un connecteur apte à être remplacé à l'extrémité de la canne et servant de support à la sonde tout en assurant les liaisons électriques entre les fils du couple thermo-électrique, de la pile électro-chimique et les conducteurs de la canne.

Le pyromètre est caractérisé en ce que le culot (30) de la sonde (10) comporte un passage tubulaire axial (46) présentant une fente latérale (48) le long de laquelle passe un conducteur (50) relié au fil en platine du couple thermo-électrique (12) et en ce que le connecteur (26) comporte une tige massive axiale en cuivre (52) apte à s'enficher dans ledit passage tubulaire (46) pour assurer la connexion électrique avec l'appareil de mesure (24).

Application aux pyromètres pour bains métalliques en fusion.

EP 0 071 534 A1

./...

FIG_3

PYROMETRE A IMMERSION.

La présente invention concerne un pyromètre à immersion du type comprenant une sonde destinée à être immergée dans un milieu, tel qu'un bain de métal en fusion, afin d'en mesurer sa température et éventuellement son activité en oxygène.

On connaît déjà dans la technique antérieure des pyromètres de ce type qui comprennent :

- une sonde à immerger dans le milieu dont on veut mesurer la température, cette sonde comprenant un couple thermo-électrique platine/platine rhodié,

- une canne pour porter la sonde à distance pendant la mesure, cette canne contenant des conducteurs électriques pour relier le couple thermo-électrique à un appareil de mesure, et

- un connecteur apte à être monté de façon détachable à l'extrémité de la canne et apte à servir de support à la sonde en assurant les liaisons électriques entre les fils du couple thermo-électrique et les conducteurs de la canne,

la sonde comportant un culot cylindrique creux et le connecteur étant apte à être emmanché par une extrémité dans ledit culot et à être emmanché par son autre extrémité dans la canne.

Les pyromètres à immersion de ce type comprennent généralement en outre un dispositif de mesure de l'activité de l'oxygène du milieu considéré, comportant deux électrodes supportées par la sonde, ces deux électrodes étant elles-mêmes reliées à deux conducteurs de la sonde destinés à être connectés avec deux autres conducteurs de la canne, par l'intermédiaire du connecteur.

Les pyromètres à immersion de ce type, connus dans la technique antérieure, sont généralement d'un prix de revient trop élevé.

D'autre part, ces pyromètres connus n'assurent pas toujours de façon efficace la solidarisation de la sonde sur la canne, ce qui risque de provoquer une chute de la sonde dans le milieu à mesurer.

La présente invention vise à éviter les inconvénients ci-dessus en proposant un pyromètre à immersion dont les divers éléments, et en particulier le connecteur, sont de fabrication moins coûteuse que les pyromètres de la technique antérieure et qui, de surcroît, assurent une meilleure sécurité au cours de leur utilisation.

La présente invention concerne plus particulièrement un pyromètre à immersion du type indiqué plus haut, dont la caractéristique essentielle réside dans le fait que le culot de la sonde comporte un passage tubulaire axial présentant une fente latérale le long de laquelle passe un conducteur relié au fil en platine du couple thermo-électrique et dans le fait que le connecteur comporte une tige massive axiale en cuivre apte à s'enficher dans ledit passage tubulaire pour assurer la connexion électrique avec l'appareil de mesure.

Selon une autre caractéristique de l'invention, le culot de la sonde comporte une rainure intérieure longitudinale dans laquelle passe un conducteur relié au fil en platine rhodié du couple thermo-électrique et le connecteur comporte alors une bague externe en cuivre apte à venir en contact avec ledit conducteur lorsque la sonde et le connecteur sont emmanchés.

Dans le cas ou le pyromètre comporte en outre deux électrodes de mesure de l'activité de l'oxygène du milieu à mesurer, le culot de la sonde comporte deux rainures intérieures longitudinales supplémentaires dans lesquell  passent deux conducteurs reliés respectivement aux deux électrodes, le connecteur comportant deux bagues externes supplémentaires en cuivre aptes à venir en contact avec les deux conducteurs précités, lorsque la sonde et le connecteur sont emmanchés.

Les zones de contact des conducteurs de la sonde avec ceux du connecteur sont disposées à l'intérieur du culot de la sonde, de manière à bien protéger lesdites zones de contact.

Selon une autre caractéristique de l'invention, les deux conducteurs reliés au couple thermo-électrique et les deux conducteurs reliés aux deux électrodes sont répartis à 90° à l'intérieur du culot de la sonde.

La présente invention prévoit également de munir le pyromètre à immersion d'un manchon de verrouillage présentant une extrémité apte à être vissée sur une extrémité de la canne et une extrémité opposée apte à s'encliqueter autour du culot de la sonde.

Ceci permet d'assurer un verrouillage efficace de la sonde à l'extrémité de la canne et par suite d'empêcher toute chute accidentelle de la sonde dans le milieu à mesurer.

Selon une autre caractéristique de l'invention, le manchon de verrouillage comporte un épaulement interne apte à venir en appui sur un épaulement externe du connecteur, afin de supprimer les jeux mécaniques entre la canne, le connecteur et le manchon.

L'extrémité du manchon de verrouillage destinée à s'encliqueter autour du culot de la sonde comporte avantageusement une bague en acier à ressort logée dans une rainure annulaire intérieure du manchon et apte à coopérer avec un bossage externe du culot de la sonde.

D'autres caractéristiques et avantages de l'invention seront mieux compris à la lecture de la description qui suit et qui se réfère aux dessins annexés sur lesquels :

    - la figure 1 est une vue en élévation représentant la canne, le connecteur et la sonde d'un pyromètre à immersion selon l'invention, à l'état séparé ;

    - la figure 2 est une vue en élévation du manchon de verrouillage destiné à équiper le pyromètre de la figure 1 ;

    - la figure 3 est une vue en coupe axiale partielle du pyromètre de la figure 1 à l'état assemblé et muni du manchon de verrouillage de la figure 2 ;

    - la figure 4 est une vue en coupe prise suivant la ligne IV-IV du culot de la sonde du pyromètre de la figure 3 ; et

    - la figure 5 est une vue d'extrémité du connecteur du pyromètre de la figure 3.

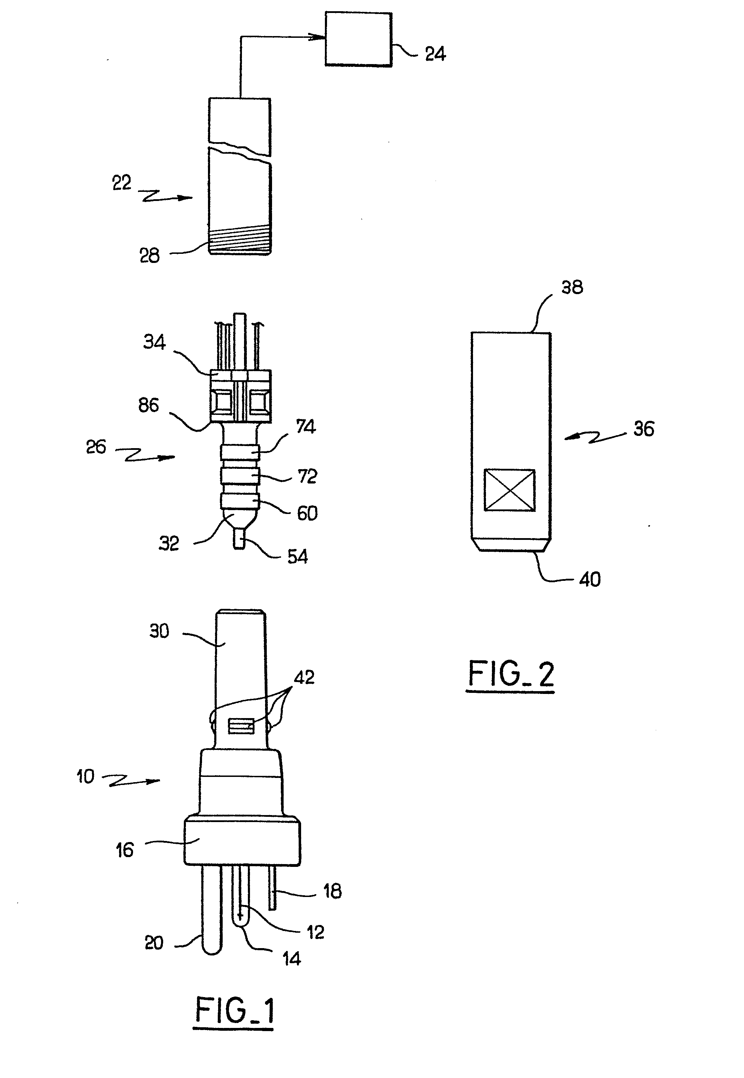

    On a représenté sur la figure 1 un pyromètre à immersion selon l'invention comprenant une sonde 10 à immerger dans le milieu, par exemple un bain de métal en fusion tel que de l'acier en fusion, dont on veut mesurer la température, cette sonde comprenant un couple thermo-électrique 12 platine/platine rhodié logé à l'intérieur d'un tube 14 porté par un support réfractaire 16 de la sonde en matière plastique remplie de ciment réfractaire. Cette sonde comporte en outre un dispositif de mesure de l'activité de l'oxygène du milieu considéré. Ce dispositif comporte une électrode 18 en molybdène et une électrode en zircone 20 contenant du molybdène + de l'oxyde de molybdène.

    Le pyromètre comporte en outre une canne 22 pour porter la sonde 10 à distance pendant la mesure, cette canne contenant des conducteurs électriques pour relier le couple thermo-électrique 12, ainsi que les électrodes 18 et 20, à un appareil de mesure 24.

Le pyromètre comporte par ailleurs un connecteur 26 apte à être monté de façon détachable à l'extrémité inférieure 28 de la canne 22 et apte à servir de support à la sonde 10 en assurant les liaisons électriques entre les fils du couple thermo-électrique et les fils des deux électrodes 18 et 20 d'une part et les conducteurs de la canne d'autre part.

Le connecteur 26 comporte une extrémité inférieure 32 apte à être emmanchée à l'intérieur du culot cylindrique creux 30 de la sonde 10 et une extrémité supérieure 34 apte à être emmanchée à l'intérieur de l'extrémité 28 de la canne 22. Lorsque la canne, le connecteur et la sonde sont assemblés, la liaison électrique est établie entre les deux fils du couple thermo-électrique 12 et les deux électrodes de la sonde, d'une part, et les quatre conducteurs de la canne, d'autre part, ces quatre conducteurs étant reliés à l'appareil de mesure 24. Pour améliorer l'assemblage de ces quatre éléments, l'invention prévoit de munir le pyromètre d'un manchon de verrouillage 36 tel que représenté sur la figure 2. Ce manchon de verrouillage 36 est destiné à recevoir le connecteur 26 et présente une extrémité supérieure 38 destinée à être vissée autour de l'extrémité 28 de la canne et une extrémité inférieure 40 destinée à être encliquetée sur des bossages 42 prévus à l'extérieur du culot 30 de la sonde 10.

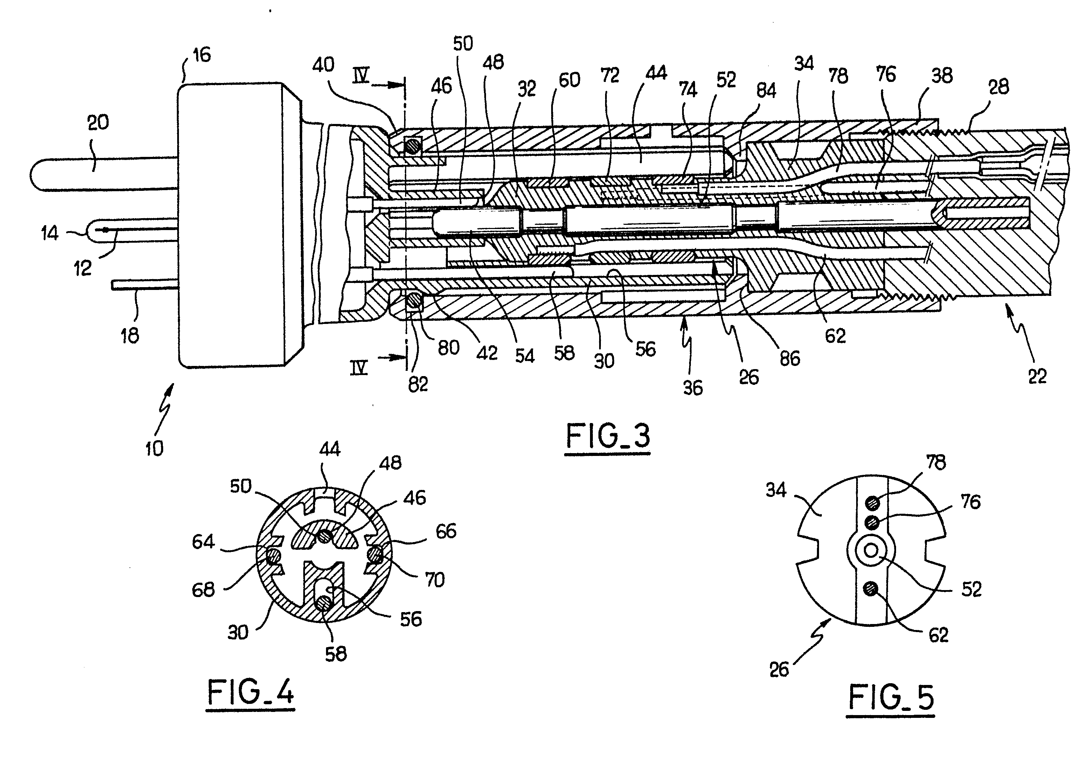

On se réfèrera maintenant plus particulièrement aux figures 3 à 5 pour décrire les caractéristiques du pyromètre de l'invention.

Le culot 30 de la sonde 10 est un culot fendu qui présente une fente longitudinale 44. A l'intérieur du culot 30 est prévu un passage tubulaire axial 46

présentant une fente latérale 48 le long de laquelle passe un conducteur 50 relié au fil en platine du couple thermo-électrique 12. Le connecteur 26 comporte quant à lui, une tige massive axiale en cuivre 52 qui présente une extrémité 54 apte à s'enficher dans le passage tubulaire 46 pour assurer la connexion avec le conducteur 50 et par conséquent avec l'appareil de mesure 24. Le culot 30 de la sonde 10 comporte par ailleurs une rainure intérieure longitudinale 56 dans laquelle passe un conducteur 58 relié au fil en platine rhodié du couple thermo-électrique 12. Le connecteur 26 comporte, quant à lui, une bague externe 60, disposée à proximité immédiate de l'extrémité 54 de la tige 52 et autour de l'extrémité inférieure 32 du connecteur 26. Cette bague 60 est destinée à venir en contact avec le conducteur 58 lorsque la sonde et le connecteur sont emmanchés. La bague 60 est par ailleurs reliée à un conducteur 62 dont l'autre extrémité comporte une connexion (non représentée) destinée à être reliée à une autre connexion (non représentée) de la canne 22.

Le culot 30 de la sonde comporte par ailleurs deux rainures intérieures longitudinales supplémentaires 64 et 66 dans lesquelles passent respectivement deux conducteurs 68 et 70 reliés respectivement aux deux électrodes 18 et 20. L'extrémité 32 du connecteur comporte deux bagues externes supplémentaires 72 et 74 en cuivre aptes à venir en contact avec les deux conducteurs 68 et 70 lorsque la sonde et le connecteur sont emmanchés. Bien entendu, les conducteurs 58, 68 et 70 présentent des zones de contact disposées à des hauteurs différentes du culot 30 de manière à pouvoir venir en contact respectivement avec les bagues 60, 72 et 74 lorsque la sonde et le connecteur sont emmanchés. Les zones de contact se trouvent alors à l'intérieur du culot de la sonde en offrant ainsi une meilleure protection vis à vis des agents extérieurs.

Comme on peut le remarquer sur la figure 4, les deux conducteurs 48 et 58 reliés au couple thermo-électrique 12 et les deux conducteurs 68 et 70 reliés aux deux électrodes sont répartis à 90° à l'intérieur du culot de la sonde.

Les bagues 72 et 74 sont reliées respectivement à des conducteurs 76 et 78 qui se terminent par des connexions aptes à coopérer avec des connexions correspondantes prévues à l'intérieur de l'extrémité 28 de la canne 22. Comme on le remarquera plus particulièrement sur la figure 5, les connexions des conducteurs 62, 76 et 78 sont alignées avec la connexion de la tige axiale 52.

Le manchon de verrouillage 36, qui est réalisé par exemple en acier, présente une extrémité filetée 38 destinée à être vissée autour de l'extrémité filetée 28 de la canne 22 et une extrémité opposée 40 munie d'un organe d'encliquetage. Cet organe d'encliquetage est constitué par une bague en acier à ressort 80 logée dans une rainure annulaire intérieure 82 du manchon 36 et apte à coopérer avec les bossages externes 42 précités. Dans l'exemple de réalisation représenté, ces bossages sont au nombre de quatre, répartis régulièrement autour du culot 30.

Le manchon de verrouillage 36 comporte par ailleurs un épaulement intérieur 84 apte à venir en appui contre un épaulement externe 86 du connecteur 26 de manière à supprimer les jeux entre le connecteur et le manchon lorsque ce dernier est vissé sur la canne.

Bien entendu, l'invention n'est pas limitée au mode de réalisation particulièrement décrit et représenté et on peut imaginer d'autres variantes de réalisation sans sortir du cadre de l'invention.

Ainsi, il est parfaitement possible de réaliser un pyromètre selon l'invention ne comportant qu'un couple thermo-électrique, auquel cas le culot 30 de la sonde 10 ne comportera que les deux conducteurs 48 et 58 et le connecteur 26 ne comportera que la bague 60 et la tige axiale 52.

Cependant, l'intérêt d'un tel connecteur muni de 4 contacts réside dans le fait que l'on a la possibililité, avec une canne équipée de ce connecteur et du manchon précédent, de procéder à des mesures, soit de température seule, soit de température plus oxygène, soit de température et de teneur en carbone.

REVENDICATIONS

1. Pyromètre à immersion du type comprenant :

- une sonde (10) à immerger dans le milieu dont on veut mesurer la température, cette sonde comprenant un couple thermo-électrique platine/platine rhodié (12),

- une canne (22) pour porter la sonde (10) à distance pendant la mesure, cette canne contenant des conducteurs électriques pour relier le couple thermo-électrique 12 à un appareil de mesure (24), et

- un connecteur (26) apte à être monté de façon détachable à l'extrémité (28) de la canne (22) et apte à servir de support à la sonde (10) en assurant les liaisons électriques entre les fils du couple thermo-électrique (12) et les conducteurs de la canne (22), la sonde (10) comportant un culot cylindrique creux (30) et le connecteur (26) étant apte à être emmanché par une extrémité (32) dans ledit culot (30) et à être emmanché par son autre extrémité (34) dans la canne (22), caractérisé en ce que le culot (30) de la sonde (10) comporte un passage tubulaire axial (46) présentant une fente latérale (48) le long de laquelle passe un conducteur (50) relié au fil en platine du couple thermo-électrique (12) et en ce que le connecteur (26) comporte une tige massive axiale en cuivre (52) apte à s'enficher dans ledit passage tubulaire (46) pour assurer la connexion électrique avec l'appareil de mesure (24).

2. Pyromètre à immersion selon la revendication 1, caractérisé en ce que le culot (30) de la sonde (10) comporte une rainure intérieure longitudinale (56) dans laquelle passe un conducteur (58) relié au fil en platine rhodié du couple thermo-électrique (12) et en ce que le connecteur (26) comporte une bague externe (60) en cuivre apte à venir en contact avec ledit conducteur (58) lorsque la sonde (10) et le connecteur (26) sont emmanchés.

3. Pyromètre selon l'une des revendications 1 et 2, dans lequel la sonde (10) comporte deux électrodes (18,20) de mesure de l'activité de l'oxygène, caractérisé en ce que le culot (30) de la sonde (10) comporte deux rainures intérieures longitudinales supplémentaires (64, 66) dans lesquelles passent deux conducteurs (68, 70) reliés respectivement aux deux électrodes (18,20) et en ce que le connecteur (26) comporte deux bagues externes supplémentaires (72,74) en cuivre aptes à venir en contact avec les deux conducteurs précités (68,70) lorsque la sonde (10) et le connecteur (26) sont emmanchés.

4. Pyromètre selon l'une des revendications 1 à 3, caractérisé en ce que les zones de contact des conducteurs (50, 58, 68, 70) de la sonde (10) avec ceux (52,60, 72, 74) du connecteur (26) sont à l'intérieur du culot (30) de la sonde (10).

5. Pyromètre selon la revendication 3, caractérisé en ce que les deux conducteurs (50,58) reliés au couple thermo-électrique (12) et les deux conducteurs (68, 70) reliés aux deux électrodes (18, 20) sont répartis à 90° à l'intérieur du culot (30) de la sonde (10).

6. Pyromètre selon l'une des revendications 1 à 5, caractérisé en ce qu'il comporte un manchon de verrouillage (36) présentant une extrémité (38) apte à être vissée sur l'extrémité (28) de la canne (22) et une extrémité opposée (40) apte à s'encliqueter autour du culot (30) de la sonde (10).

7. Pyromètre selon la revendication 6, caractérisé en ce que le manchon de verrouillage (36) comporte un épaulement interne (84) apte à venir en appui sur un épaulement externe (86) du connecteur (26).

8. Pyromètre selon la revendication 6, caractérisé en ce que l'extrémité (40) du manchon de verrouillage (36) destinée à s'encliqueter autour du culot (30) de la sonde (10) comporte une bague (80) en acier à ressort logée dans une rainure annulaire intérieure (82) du manchon (36) et apte à coopérer avec un bossage externe (42) du culot (30) de la sonde (10).

24

22

28

34

86

26

74

72

60

32

54

38

36

40

FIG_2

30

42

10

16

18

12

20

14

FIG_1

FIG_3

FIG_4

FIG_5

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| | --- | | G 01 K 7/02 |
| X | FR-A-2 380 650 (MANNESMANN A.G. ET GUSTAV KOLB, FABRIK ELEKTROKERAMISCHE ARTIKEL) * figures 2,3,4,5,6,7; titre; page 7, ligne 12 - page 10, ligne 14 * | 1-3,5 | H 01 R 23/26 |
| | --- | | |
| A | US-A-3 907 395 (W.H. FLANAGAN) * figures 1,2,4; colonne 2, ligne 9 - colonne 3, ligne 9; colonne 4, lignes 1-9 * | 6-8 | |
| | ----- | | |

| DOMAINES TECHNIQUES RECHERCHES (Int. Cl. ³) |
|---|
| G 01 K H 01 R |

Le présent rapport de recherche a été établi pour toutes les revendications

| Lieu de la recherche | Date d'achèvement de la recherche | Examinateur |
|---|---|---|
| LA HAYE | 05-11-1982 | VISSER F.P.C. |